# EUROPEAN PATENT APPLICATION

(11) **EP 4 282 952 A1**
(43) Date of publication of application: **29.11.2023**
(21) Application number: 22760080.6
(22) Date of filing: 24.02.2022
(51) Int. Cl.: C12N 5/071, A61K 35/44, A61K 35/28, A61K 38/18, A61P 9/10, A61K 48/00

(54) **METHOD FOR PREPARING BONE-MARROW-DERIVED ENDOTHELIAL PROGENITOR CELLS HAVING INCREASED VASCULOGENESIS BY USING PDGF-BB, AND METHOD FOR PREPARING CELL THERAPEUTIC AGENT FOR TREATING ANANGIOPLASIA-RELATED DISEASES BY USING METHOD**

(30) Priority: 26.02.2021 KR 20210026420
(71) Applicant: UNIVERSITY-INDUSTRY COOPERATION GROUP OF KYUNG HEE UNIVERSITY, Yongin-si, Gyeonggi-do 17104 (KR); Elphis Cell Therapeutics, Seoul 02455 (KR)
(72) Inventor: SON, Youngsook, Seoul 06305 (KR); KIM, Suna, Yongin-si Gyeonggi-do 17106 (KR); KIM, Doyoung, Namyangju-si Gyeonggi-do 10333 (KR)
(74) Representative: Isarpatent
(86) International application number: PCT/KR2022/002713
(87) International publication number: WO 2022/182164

(57) **Abstract**

The present invention relates to a method for preparing bone marrow-derived endothelial progenitor cells having increased vasculogenesis by using PDGF-BB, and a method for preparing a cell therapeutic agent for treating anangioplasia-related diseases by using the method. Particularly, the present invention relates to: a method for preparing endothelial progenitor cells having increased vasculogenesis, comprising a step of treating isolated endothelial progenitor cells with PDGF-BB and culturing same; a composition for stimulating vasculogenesis, comprising endothelial progenitor cells and PDGF-BB as active ingredients; a method for preparing a cell therapeutic agent for treating anangioplasia-related diseases, comprising a step of mixing isolated endothelial progenitor cells, mesenchymal stem cells and PDGF-BB; and a cell therapeutic agent for treating anangioplasia-related diseases, prepared by the method.

## Description

### [Technical Field]

The present invention relates to a method for preparing bone marrow-endothelial progenitor cells having increased vasculogenesis by using PDGF-BB, and a method for preparing a cell therapeutic agent for treating anangioplasia-related diseases by using the method.

### [Background Art]

Endothelial progenitor cells (EPCs) are cells that were first identified in peripheral blood and stimulate angiogenesis.

New blood vessels are generated by angiogenesis, arteriogenesis and vasculogenesis, and the angiogenesis is related to the proliferation and migration of endothelial cells that grow from already existing mature endothelial cells. The arteriogenesis is a process of remodeling existing arteriolar connections into collateral vessels, and the vasculogenesis proceeds through a process in which endothelial progenitor cells differentiate into mature endothelial cells. Therefore, the endothelial progenitor cells circulating from the bone marrow migrate to a vascular damage area, and are involved in neovascularization through direct insertion into newly formed blood vessels or secretion of various angiogenesis and trophic factors.

Therefore, the endothelial progenitor cells are attracting attention as potential targets in the field of regenerative medicine and for therapeutic purposes through reangiogenesis. Accordingly, studies have been conducted to increase the function of the endothelial progenitor cells, and for example, studies on the preparation of recombinant EPCs obtained by modifying *ex vivo* genes have been conducted, and a technique for increasing the pro-angiogenic capacity of EPCs using a vascular endothelial growth factor (VEGF) or hypoxiainducible factor-1α has been studied.

However, in the conventional method, when the endothelial progenitor cells are applied to the vessel damage area, there is a problem in that a desired therapeutic effect is not obtained due to the limitations of low transplantation rate and low activation of cells due to a damaged tissue environment.

In addition, a conventional treatment method using endothelial progenitor cells is applied to revascularization treatment by concentrating CD34+ cells simply isolated from patient's blood by leukapheresis and FACS methods through a minimally manipulated cell therapy to be transplanted to a myocardial infarction area and the like where vasculogenesis is required. Such a therapy is not used for patients as a new medical technology because patient-specific changes are large in the number (frequency of endothelial progenitor cells in the blood: 0.02 to 0.0002%) and activity of endothelial progenitor cells, and a constant increase in cardiac ejection fraction after transplantation (e.g., contractility (LVEF, left ventricular ejection fraction) increased from about 50% before treatment to about 55% after treatment) is not realized.

Therefore, it is necessary to develop a new technology that can be usefully used as a cell therapeutic agent for anangioplasia-related diseases by effectively increasing vasculogenesis of the endothelial progenitor cells.

### [Disclosure]

### [Technical Problem]

Therefore, the present inventors analyzed the cell characteristics of endothelial progenitor cells isolated from the bone marrow for each culture passage and evaluated the revascularization capacity, confirmed that endothelial progenitor cells were treated with PDGF-BB to effectively improve the vasculogenesis, cell migration, cell proliferation and cell viability of endothelial progenitor cells, and further confirmed that there was a more improved vasculogenesis effect when mixed cells of endothelial progenitor cells and mesenchymal stem cells were treated with PDGF-BB and co-cultured. Accordingly, the present inventors completed the present invention by establishing a method for preparing a novel cell therapeutic agent capable of effectively improving vasculogenesis of endothelial progenitor cells.

Accordingly, an object of the present invention is to provide a method for preparing endothelial progenitor cells having increased vasculogenesis using platelet-derived growth factor-BB (PDGF-BB).

Another object of the present invention is to provide a composition for stimulating vasculogenesis for treating anangioplasia-related diseases, including isolated endothelial progenitor cells (EPCs), mesenchymal stem cells (MSCs), and platelet-derived growth factor-BB (PDGF-BB) as active ingredients.

Yet another object of the present invention is to provide a method for preparing a cell therapeutic agent for treating anangioplasia-related diseases, including a step of mixing isolated endothelial progenitor cells (EPCs), mesenchymal stem cells (MSCs), and platelet-derived growth factor-BB (PDGF-BB).

Yet another object of the present invention is to provide a cell therapeutic agent for treating anangioplasia-related diseases, prepared by the method of the present invention.

### [Technical Solution]

In order to achieve the object of the present invention, an aspect of the present invention provides a method for preparing endothelial progenitor cells having increased vasculogenesis, including a step of treating and culturing isolated endothelial progenitor cells (EPCs) with platelet-derived growth factor-BB (PDGF-BB).

In one embodiment of the present invention, the endothelial progenitor cells may be human bone marrow-endothelial progenitor cells (BM-EPCs).

In one embodiment of the present invention, the endothelial progenitor cells treated with PDGF-BB may have increased cell migration, increased cell viability, increased cell proliferation, and increased expression of an extra-matrix protein laminin β1.

Another aspect of the present invention provides a composition for stimulating vasculogenesis for treating anangioplasia-related diseases including isolated endothelial progenitor cells (EPCs), mesenchymal stem cells (MSCs), and platelet-derived growth factor-BB (PDGF-BB) as active ingredients.

In one embodiment of the present invention, the endothelial progenitor cells may be human bone marrow-endothelial progenitor cells (BM-EPCs).

Another aspect of the present invention provides a method for preparing a cell therapeutic agent for treating anangioplasia-related diseases including a step of mixing isolated endothelial progenitor cells (EPCs), mesenchymal stem cells (MSCs), and platelet-derived growth factor-BB (PDGF-BB).

In one embodiment of the present invention, the isolated endothelial progenitor cells and the mesenchymal stem cells may be mixed in a cell number ratio of 1 : 1 to 2 : 1.

In one embodiment of the present invention, the endothelial progenitor cells are human bone marrow-endothelial progenitor cells, and the mesenchymal stem cells may be human bone marrow-mesenchymal stem cells.

In one embodiment of the present invention, the anangioplasia-related disease may be selected from the group consisting of ischemic disease, diabetic ulcer, gangrene, obstructive vascular disease, cardiovascular disease and ischemia.

In one embodiment of the present invention, the ischemic disease may be selected from the group consisting of ischemic myocardial infarction, ischemic heart disease, ischemic vascular disease, ischemic eye disease, ischemic renal failure, ischemic retinopathy, ischemic stroke and ischemic lower limb disease.

Another aspect of the present invention provides a cell therapeutic agent for treating anangioplasia-related diseases, prepared by the method of the present invention.

In one embodiment of the present invention, the cell therapeutic agent may be mixed with human bone marrow-endothelial progenitor cells and human bone marrow-mesenchymal stem cells in a cell number ratio of 1 : 1 to 2 : 1 and have vasculogenesis.

In one embodiment of the present invention, the cell therapeutic agent may be for intramedullary administration, intravenous administration, subcutaneous administration, intramuscular administration or intraperitoneal administration.

In one embodiment of the present invention, the anangioplasia-related disease may be selected from the group consisting of ischemic disease, diabetic ulcer, gangrene, obstructive vascular disease, cardiovascular disease and ischemia.

In one embodiment of the present invention, the ischemic disease may be selected from the group consisting of ischemic myocardial infarction, ischemic heart disease, ischemic vascular disease, ischemic eye disease, ischemic renal failure, ischemic retinopathy, ischemic stroke and ischemic limb disease.

Another aspect of the present invention provides a method for preventing or treating anangioplasia-related diseases, including a step of administering the composition for stimulating vasculogenesis to a subject.

Another aspect of the present invention provides a method for preventing or treating anangioplasia-related diseases, including a step of administering the cell therapeutic agent prepared by the preparing method to a subject.

### [Advantageous Effects]

According to the present invention, it has been identified that endothelial progenitor cells treated with PDGF-BB have increased vasculogenesis, cell migration, cell viability and cell proliferation. Furthermore, when the mixed cells of endothelial progenitor cells and mesenchymal stem cells are treated with PDGF-BB, further increased vasculogenesis has been confirmed. Ultimately, the present invention has an effect of providing a novel cell therapeutic agent for effective treatment of anangioplasia-related diseases and a method for preparing the same.

### [Description of Drawings]

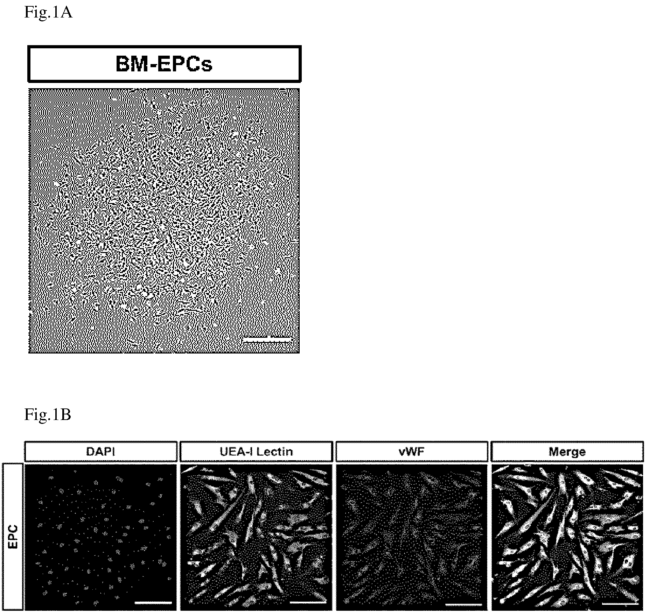

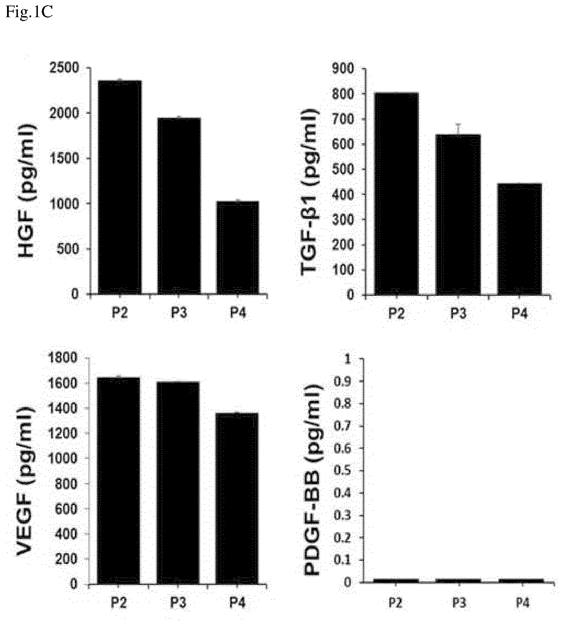

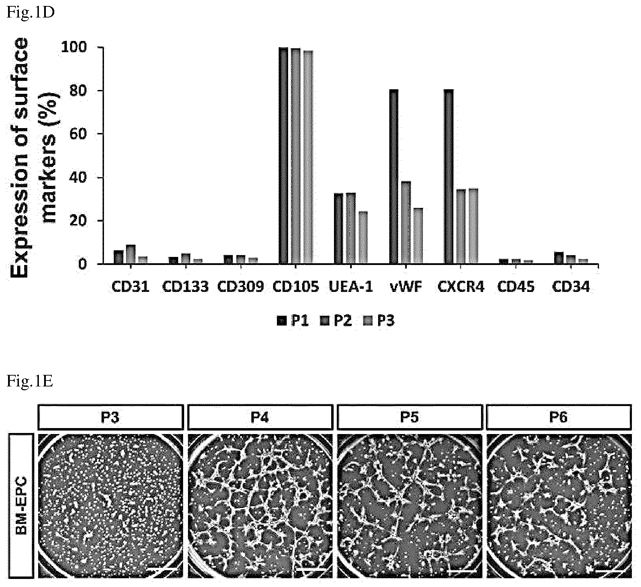

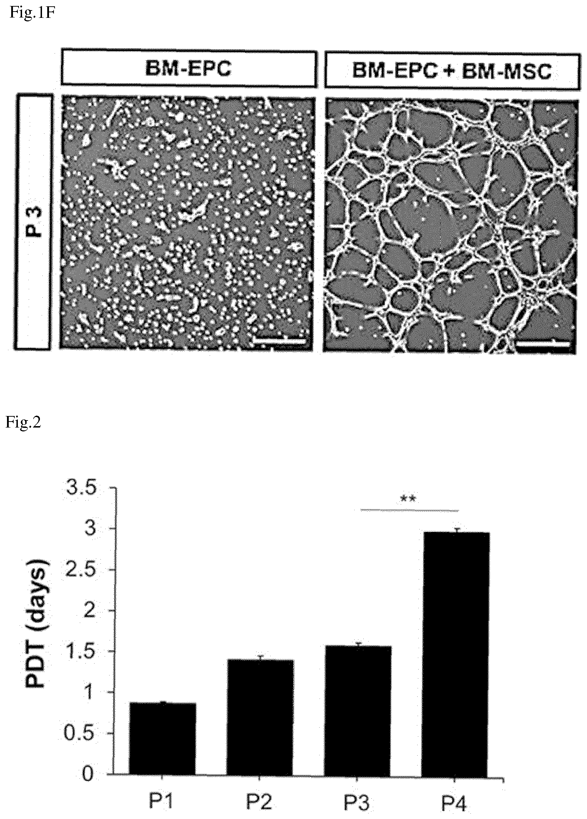

FIG. 1 is a result of analyzing the characteristics of human bone marrow-endothelial progenitor cells (BM-EPCs) according to the present invention. FIG. 1A confirms the colony formation capacity of human BM-EPCs before passage (P0), FIG. 1B illustrates the observation of marker expression (FITC-UEA-I binding: green, vWF: red) of endothelial progenitor cells by performing double fluorescence staining, FIG. 1C illustrates measurement of the concentration of growth factors contained in a BM-EPC medium conditioned for each of passages 2 to 4 (P2 to P4), FIG. 1D illustrates analysis of cells expressing cell surface markers in passages 1 to 3 (P1 to P3) by a flow cytometer, FIG. 1E illustrates a result of analyzing the tube formation capacity of BM-EPCs in passages 3 to 6 (P3 to P6), and FIG. 1F illustrates a result of analyzing the tube formation capacity of BM-EPCs in passage 3 according to the presence or absence of human bone marrow-mesenchymal stem cells (BM-MSC).
FIG. 2 is a result of measuring the population doubling time (PDT) of BM-EPCs according to a subculture.

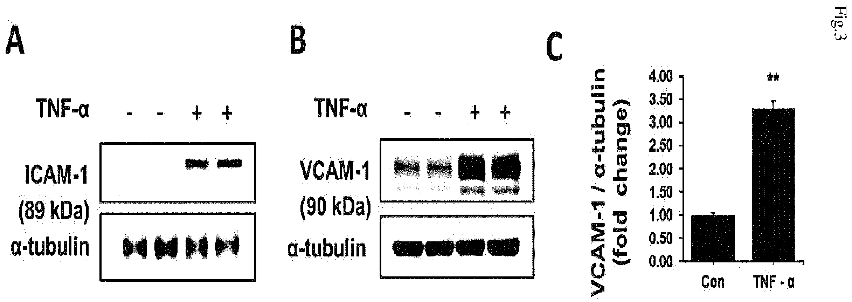

FIG. 3 is a result of Western blot analysis of changes in expression of inflammatory adhesion factors in BM-EPCs by TNF-α treatment.

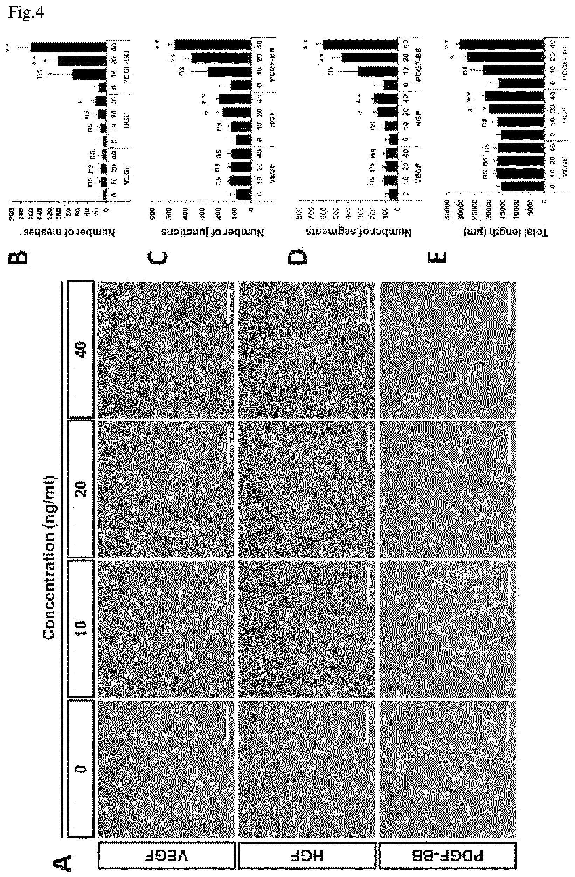

FIG. 4 is a result of analyzing the vasculogenesis of endothelial progenitor cells (BM-EPCs) for each treatment concentration of VEGF, HGF and PDGF-BB *in vitro,* in which the left diagram illustrates an image result of confirming the vasculogenesis of BM-EPCs after 4 hours after treating each growth factor, and the right graph shows a result of quantitatively measuring the number of meshes, the number of junctions, the number of segments, and a total length.

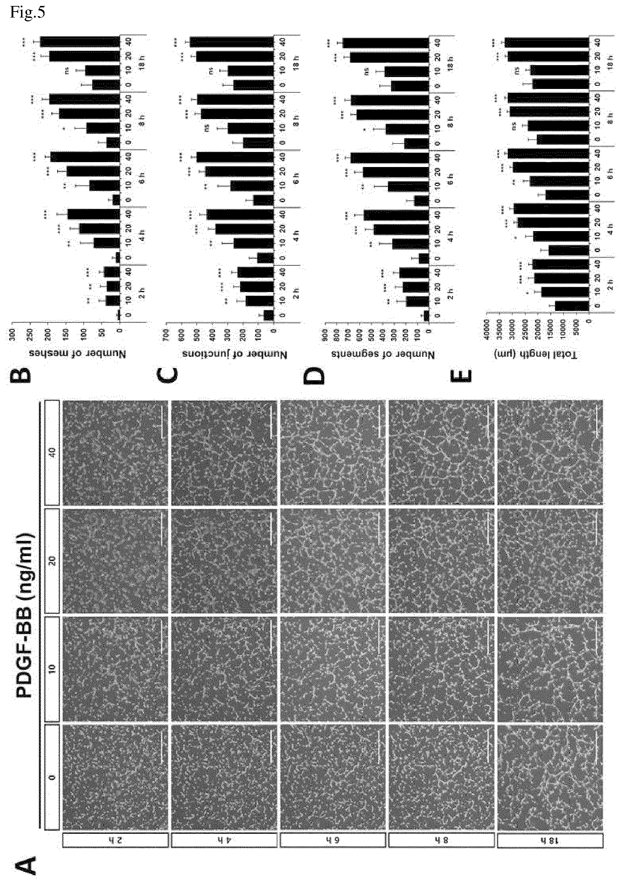

FIG. 5 is a result of analyzing vasculogenesis of endothelial progenitor cells (BM-EPCs) by treatment concentration and time of PDGF-BB.

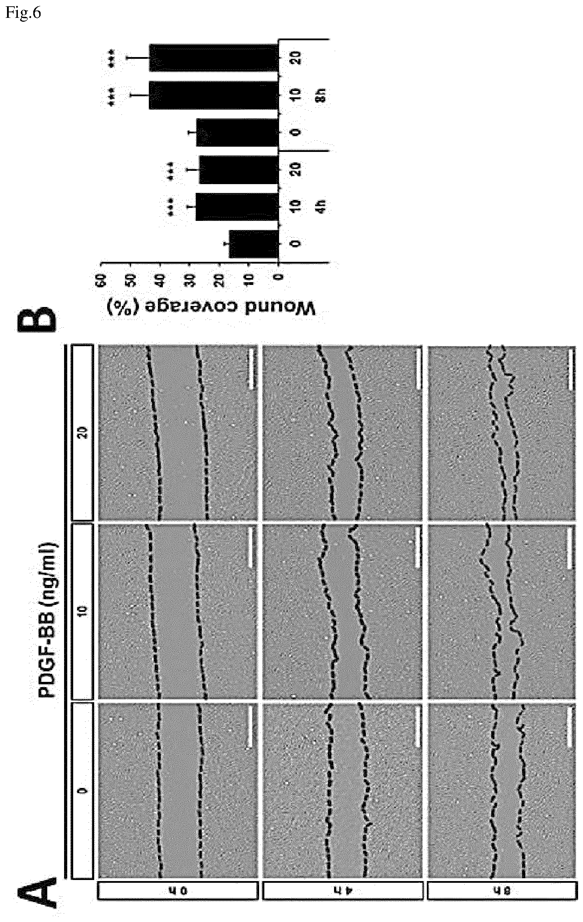

FIG. 6 is a result of confirming an increase in migration capacity of endothelial progenitor cells (BM-EPCs) by treatment concentration and time of PDGF-BB, which confirms the degree of wound recovery by cell migration of endothelial progenitor cells according to PDGF-BB treatment after scratching monolayers of the endothelial progenitor cells.

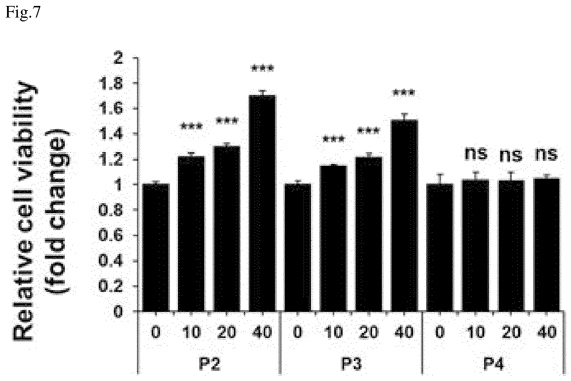

FIG. 7 is a result of confirming increased cell viability (cell activity) of endothelial progenitor cells (BM-EPCs) according to the treatment of PDGF-BB, which is a result of analyzing cell viability by WST assay after treating endothelial progenitor cells by passages P2, P3, and P4 with PDGF-BB for each concentration.

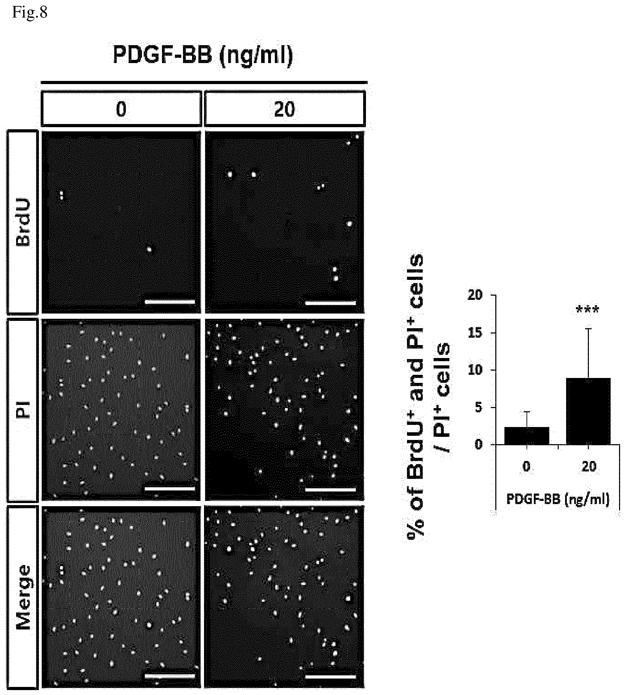

FIG. 8 is a result of confirming the cell proliferation activity of endothelial progenitor cells (BM-EPCs) according to the treatment with PDGF-BB by a BrdU assay method.

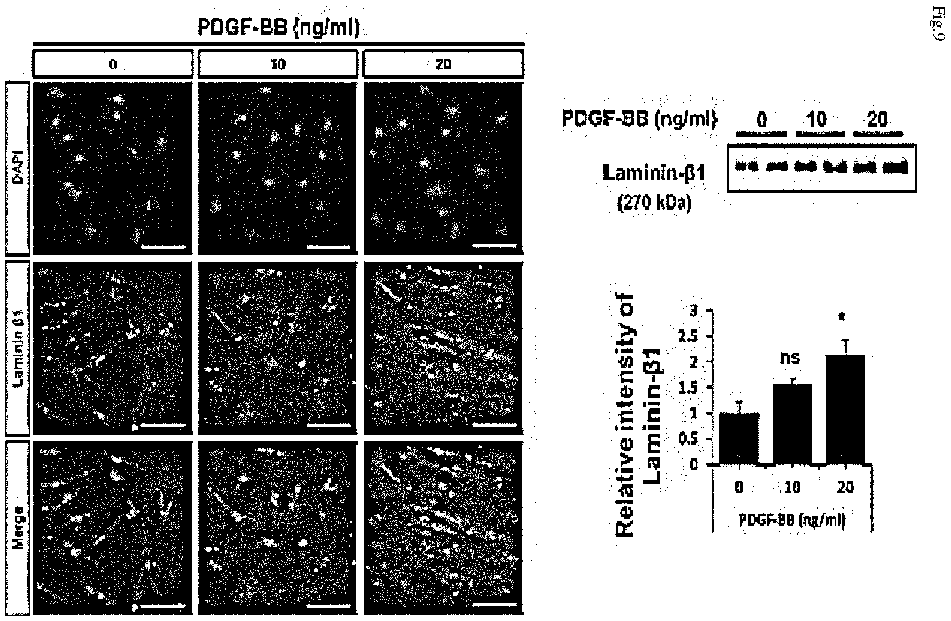

FIG. 9 is a result of analyzing the expression change of extracellular matrix proteins of endothelial progenitor cells (BM-EPCs) according to the treatment of PDGF-BB, which is a result of analyzing the expression level of Laminini-β1 according to a treatment concentration of PDGF-BB by immunofluorescence staining and Western blotting.

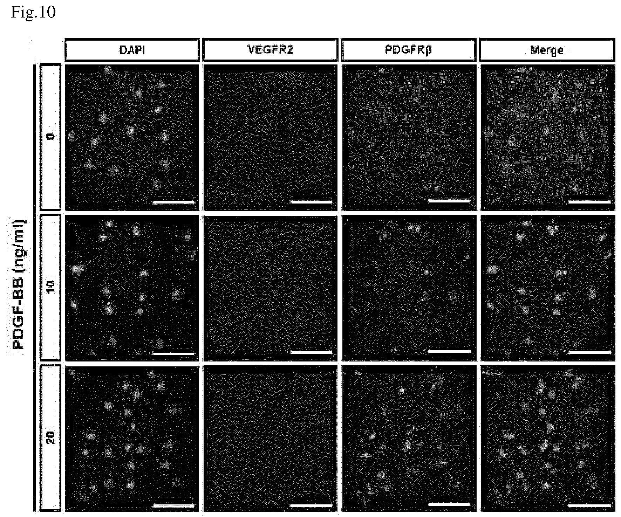

FIG. 10 is a result of confirming an increase in PDGFRβ internalization of endothelial progenitor cells (BM-EPCs) according to the treatment of PDGF-BB, which is a result of observing VEGFR2 and PDGFRβ in endothelial progenitor cells by immunofluorescence staining after 24 hours after treating the PDGF-BB for each concentration.

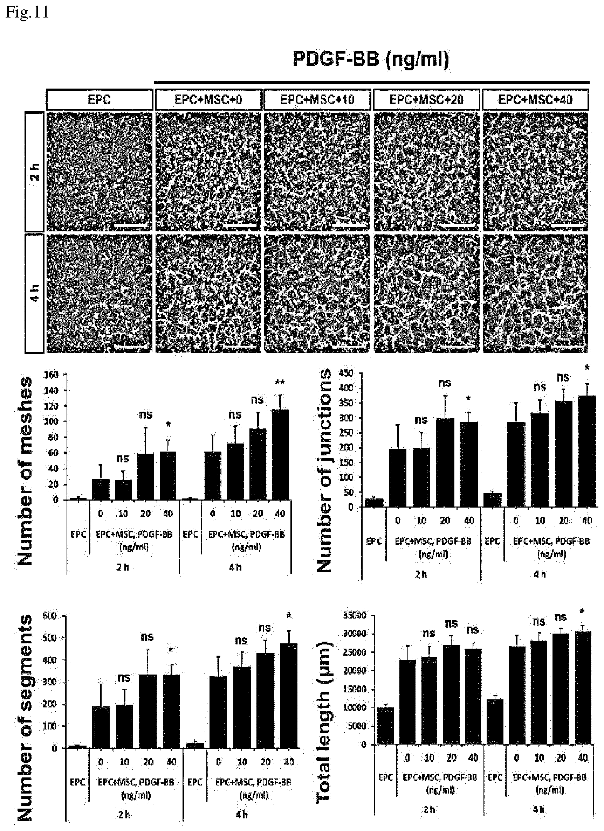

FIG. 11 is a result of analyzing vasculogenesis of endothelial progenitor cells and mesenchymal stem cells according to the treatment of PDGF-BB, and photographs of observing vasculogenesis according to treatment concentration and treatment time of PDGF-BB while co-culturing endothelial progenitor cells and bone marrow-mesenchymal stem cells through a microscope. In addition, the following graphs show results of quantitatively measuring the number of meshes, the number of junctions, the number of segments, and the total length for each experimental group.

### [Best Mode for the Invention]

The present invention provides a method for preparing endothelial progenitor cells having increased vasculogenesis, a novel cell therapeutic agent for treating anangioplasia-related diseases using the same, and a method for preparing the same.

Blood vessels are generated by proliferation of endothelial cells. Vasculogenesis occurs in the early stages of development, and thereafter, angiogenesis, in which new blood vessels are formed from existing capillaries, occurs. The angiogenesis is allowed in the body only in special cases, such as when a wound is healed, but is strictly inhibited otherwise.

In addition, the endothelial progenitor cells (EPCs) are cells derived from bone marrow and cells that may mature into endothelial cells forming blood vessels. Endothelial progenitor cells may be identified by detecting specific markers CD31, CD34, CD133, CD144 and CD309 in whole blood. The endothelial progenitor cells play an important role in re-endothelialization and neovascularization.

On the other hand, when vasculogenesis occurs excessively, the excessive vasculogenesis becomes a major cause of disease aggravation, but non-vascularization also causes serious diseases. Particularly, the non-vascularization, that is, anangioplasia may cause necrosis, ulceration, and ischemia, and in this case, causes tissue or organ dysfunction. In addition, diseases such as arteriosclerosis, myocardial infarction, and angina are also caused by poor blood supply.

Therefore, it is very important to reduce tissue damage caused by induction of hypoxia or hyponutrition due to anangioplasia (hypoplasia), and to induce or stimulate neovascularization for smooth tissue regeneration.

In particular, angiogenesis needs to necessarily accompany an essential wound healing process for regenerating injured skin tissue. In the early stages of injury, an inflammatory response occurs due to cell necrosis and vascular destruction, and after this inflammatory response, a series of processes are performed so that biological mediators such as kallikrein, thrombin, and plasmin are formed along with devascularization of blood components, activation of platelets, and blood coagulation.

On the other hand, as a method for treating diseases caused by such anangioplasia, a method of utilizing endogenous stem cells *in vivo* to regenerate vascular damage and a method of using endothelial progenitor cells or endothelial progenitor cell-like cells have been applied. However, so far, no significant efficacy has been identified, and patients with underlying diseases have very low numbers and activity of endothelial progenitor cells compared to normal healthy people, so that there is a need for a technique capable of increasing the number of endothelial progenitor cells and increasing vasculogenesis.

Accordingly, the present inventors studied a method capable of increasing the vasculogenesis of endothelial cells as an effective cell therapeutic agent for treating anangioplasia-related diseases, and as a result, identified that when endothelial progenitor cells were treated and cultured with platelet-derived growth factor-BB (PDGF-BB), not only the vasculogenesis of the endothelial progenitor cells treated with PDGF-BB was increased, but also the cell activity and cell proliferation were also significantly improved.

Therefore, the present invention may provide a method for preparing endothelial progenitor cells having increased vasculogenesis, including a step of treating and culturing isolated endothelial progenitor cells (EPCs) with platelet-derived growth factor-BB (PDGF-BB).

In the present invention, the endothelial progenitor cells may be human bone marrow-endothelial progenitor cells (BM-EPCs).

In addition, the treating of the isolated endothelial progenitor cells with PDGF-BB may be performed by adding PDGF-BB to the culture medium of the endothelial progenitor cells, and at this time, as the endothelial progenitor cells, endothelial progenitor cells subcultured for passages 1 to 3 (P1 to P3) may be used, preferably endothelial progenitor cells subcultured for passages 2 to 3 (P2 to P3) may be used.

The "subculturing", as one of cell proliferation methods, means that the cells are periodically transplanted into a new medium every 5 to 7 days to continue the passages of the cells, and specifically, "passage" means the growth of cells from an initial seed culture in a culture vessel to the time (confluence) when cells grow vigorously in the same culture vessel.

In one embodiment of the present invention, after treating endothelial progenitor cells with PDGF-BB for each passage, cell activity (cell viability) of the endothelial progenitor cells was analyzed, but it was shown that the cell activity increased by PDGF-BB treatment in the endothelial progenitor cells in P1 to P3 passages, whereas there was no significant difference by PDGF-BB treatment in P4 passage. Accordingly, the PDGF-BB is preferably treated on isolated endothelial progenitor cells in P1 to P3 passages.

The PDGF-BB may be treated in a culture medium at a concentration of 10 to 40 ng/ml.

The culturing may be performed in a cell culture medium used in the art, and in one embodiment of the present invention, the culturing was performed using an a-MEM medium added with 0.2% FBS.

According to an embodiment of the present invention, the culture medium of bone marrow-endothelial progenitor cells is treated with VEGF, HGF and PDGF-BB, which are angiogenesis stimulating factors, respectively, and a structure of vasculogenesis was observed. It was shown that VEGF and HGF showed an insignificant vasculogenesis effect, whereas specifically, vasculogenesis was significantly increased only in a group treated with PDGF-BB.

Through this, the present inventors confirmed that PDGF-BB may be used as a method for preparing endothelial progenitor cells having increased vasculogenesis.

In the present invention, it was confirmed through an experiment that the PDGF-BB-treated endothelial progenitor cells had characteristics of not only increased vasculogenesis, but also increased cell migration, increased cell viability, increased cell proliferation, and increased expression of an extra-matrix protein laminin β1 of the endothelial progenitor cells.

Accordingly, the present invention may provide a method for preparing endothelial progenitor cells having increased vasculogenesis including a step of treating and culturing isolated endothelial progenitor cells with PDGF-BB, and further, provide a method for increasing the number of endothelial progenitor cells including a step of treating and culturing isolated endothelial progenitor cells with PDGF-BB.

In addition, the present invention has characteristics capable of providing a composition for stimulating vasculogenesis including endothelial progenitor cells (EPCs) and platelet-derived growth factor-BB (PDGF-BB) as active ingredients.

The composition for stimulating vasculogenesis of the present invention includes the endothelial progenitor cells and PDGF-BB together as active ingredients, and as described above, has a characteristic of stimulating vasculogenesis through the action of stimulating cell migration, cell viability, and cell proliferation by endothelial progenitor cells having increased vasculogenesis by PDGF-BB.

Accordingly, the composition for stimulating vasculogenesis according to the present invention has an effect of preventing or treating anangioplasia-related diseases, and thus, the present invention may provide a pharmaceutical composition for preventing or treating anangioplasia-related diseases containing the composition for stimulating vasculogenesis as an active ingredient.

The anangioplasia-related diseases that may be prevented or treated by the composition for stimulating vasculogenesis according to the present invention are not limited thereto, but may be selected from the group consisting of ischemic diseases, diabetic ulcers, gangrene, obstructive vascular disease, cardiovascular disease and ischemia; and the ischemic disease is not limited thereto, but may include ischemic myocardial infarction, ischemic heart disease, ischemic vascular disease, ischemic eye disease, ischemic renal failure, ischemic retinopathy, ischemic stroke, and ischemic lower limb disease.

The composition for stimulating vasculogenesis or the pharmaceutical composition for preventing or treating anangioplasia-related diseases according to the present invention may include a pharmaceutically effective amount of active ingredient alone or one or more pharmaceutically acceptable carriers, excipients or diluents as a physiologically active ingredient.

Here, the "pharmaceutically effective amount" refers to an amount sufficient to exhibit a desired physiological or pharmacological activity when the physiologically active ingredient is administered to animals or humans. However, the pharmaceutically effective amount may be appropriately changed depending on the age, weight, health condition, sex, administration route and treatment period of a subject to be administered.

The "pharmaceutically acceptable" means being physiologically acceptable and generally not causing an allergic reaction, such as gastrointestinal disorder, dizziness, etc., or similar responses thereto when administered to humans. Examples of the carrier, the excipient, and the diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methyl hydroxybenzoate, propyl hydroxybenzoate, talc, magnesium stearate, and mineral oil. In addition, the pharmaceutical composition may further include fillers, anti-coagulating agents, lubricants, wetting agents, flavorings, emulsifiers, preservatives and the like.

Further, the composition of the present invention may be formulated by using a method known in the art so as to provide rapid, sustained, or delayed release of the active ingredient after administrated to mammals, and may be formulated in various forms for parenteral administration.

Representative formulations for parenteral administration are injection formulations, preferably isotonic aqueous solutions or suspensions. The injection formulations may be prepared according to techniques known in the art by using suitable dispersing or wetting agents and suspending agents. For example, the injection formulation may be formulated for injection by dissolving each ingredient in saline or buffer.

The composition according to the present invention may be administered through various routes including transdermal, subcutaneous, intravenous or intramuscular routes, and the dosage of the active ingredient may be appropriately selected according to several factors, such as a route of administration, the age, sex, and body weight of a patient, and the severity of a patient. In addition, the composition of the present invention may be administered in combination with known compounds capable of increasing the desired effect.

In addition, the present invention may provide a method for preparing a cell therapeutic agent for treating anangioplasia-related diseases, and the method includes a step of mixing isolated endothelial progenitor cells (EPCs), mesenchymal stem cells (MSCs) and platelet-derived growth factor-BB (PDGF-BB).

In the method for preparing the cell therapeutic agent of the present invention, the isolated endothelial progenitor cells and the mesenchymal stem cells may be mixed cells mixed in a cell number ratio of 1 : 1 to 2 : 1, preferably the endothelial progenitor cells and the mesenchymal stem cells may be mixed and used in a cell number ratio of 2 : 1.

The "mesenchymal stem cells (MSCs)" refer to multipotent stem cells capable of differentiating into various mesenchymal cells including bone, cartilage, fat, and muscle cells. The mesenchymal stem cells may be derived from umbilical cord, cord blood, bone marrow, fat, muscle, nerve, skin, amnion, chorion, decidua or placenta, preferably human bone marrow-mesenchymal stem cells (BM-MSCs). The mesenchymal stem cells have the capacity to regenerate damaged tissues and cells by migrating directly to a damaged area of the body and may easily proliferate *in vitro,* and have been used as useful targets in gene therapy and cell therapy as multipotent cells capable of differentiating into various cell forms.

The endothelial progenitor cells used herein may be human bone marrow-endothelial progenitor cells.

In addition, in the preparation of the cell therapeutic agent according to the present invention, pericytes may also be used instead of the mesenchymal stem cells.

The "pericyte" refers to a vascular mural cell located within the basal membrane of the microvascular system, which forms a specific local contact with the vascular endothelium. The pericytes are connective tissue cells around small blood vessels, also called Rouget cells, adventitial cells or mural cells, and known to surround 10% to 50% of the outside of the vascular endothelium. The pericyte is a thin and elongated contractile cell that surrounds around the precapillary arteriole outside the basal membrane.

The pericytes are relatively undifferentiated pluripotent cells that function to support blood vessels and may differentiate into fibroblasts, smooth muscle cells, or macrophages as needed. In addition, the pericytes play an important role in blood-brain barrier stability as well as angiogenesis, and may regulate blood flow in the microvascular system by adhesion to intravascular cells.

Therefore, the preparation of the cell therapeutic agent according to the present invention may increase the vasculogenesis of the endothelial progenitor cells by treatment with PDGF-BB, and further increase angiogenesis, vascular regeneration, and stability of blood vessel barriers through co-culture with mesenchymal stem cells or pericytes.

Accordingly, the present invention may provide not only a method for preparing a cell therapeutic agent for treating anangioplasia-related diseases, but also a cell therapeutic agent for treating anangioplasia-related diseases prepared by the method.

The cell therapeutic agent of the present invention may further include a support for accommodating the cells, preferably a biodegradable support. The biodegradable support may be a hydrogel such as fibrin glue, hyaluronic acid, gelatin, collagen, alginic acid, cellulose, pectin, chitin, polyglycolic acid, or polylactic acid, but is not limited thereto.

In addition, the cell therapeutic agent may further include a pharmaceutically acceptable carrier, and the pharmaceutically acceptable carrier may be used by mixing saline, sterile water, Ringer's solution, buffered saline, dextrose solution, maltodextrin solution, glycerol, ethanol, and at least one of these components, and if necessary, general other additives such as an antioxidant, a buffer and a bacteriostat may be added.

The cell therapeutic agent may be formulated into an injection formulation such as an aqueous solution, a suspension, or an emulsion by additionally adding a diluent, a dispersant, a surfactant, a binder, and a lubricant, and may be administered intramedullarily, intravenously, subcutaneously, intramuscularly or intraperitoneally.

In addition, the anangioplasia-related diseases, which are diseases to be treated by the cell therapeutic agent according to the present invention, may be selected from the group consisting of ischemic diseases, diabetic ulcers, gangrene, obstructive vascular diseases, cardiovascular diseases, and ischemia.

The ischemic disease is not limited thereto, but may be selected from the group consisting of ischemic myocardial infarction, ischemic heart disease, ischemic vascular disease, ischemic eye disease, ischemic renal failure, ischemic retinopathy, ischemic stroke, and ischemic lower limb disease.

In addition, the present invention provides a method for preventing or treating anangioplasia-related diseases, including a step of administering the composition for stimulating vasculogenesis to a subject.

In addition, the present invention provides a method for preventing or treating anangioplasia-related diseases, including a step of administering the cell therapeutic agent prepared by the preparing method to a subject.

### [Modes for the Invention]

Hereinafter, the components and technical features of the present invention will be described in more detail through the following Examples. However, the following Experimental Examples are merely illustrative of the contents of the present invention, but the scope of the present invention is not limited by Examples.

### <Experimental Example 1> Experimental method

### 1-1. Cell culture and EPC characteristic analysis

Human BM monocytic cells (MNCs) were purchased from STEMCELL Technologies Inc (Vancouver, Canada) and used. For EPC culture, the BM-MNCs were cultured in an endothelial growth medium-2 (EGM-2, Lonza, Basel, Switzerland) in a 100 mm dish coated with type I collagen (CellMatrix, VA, USA). The medium was changed daily until day 7 and then every 3 days. The EPCs were subcultured at 70 to 80% confluency. For MSC culture, the BM-MNCs were cultured in a mesenchymal stem cell growth medium (MSCGM, Lonza) in a 100 mm dish. The medium was replaced every 3 days and subcultured at 70 to 80% confluency.

For immunofluorescence (IF) staining, BM-EPCs (1.0 × 10⁴) were cultured on a collagen type I coated coverslip, immobilized with 3.7% formaldehyde, permeabilized with 0.2% Triton x-100 buffer for 10 minutes, and blocked with 20% normal goat serum. Ulex europaeus agglutinin (UEA)-I lectin binding was performed using fluorescein (FITC)-labeled UEA-I lectin (Sigma Aldrich, St. Louis, MO). Thereafter, a von Willebrand factor (vWF, abcam, Cambridge, England), PDGFRβ (abcam), VEGFR2 (abcam), and laminin (Santa Cruz, California, USA) were used as primary antibodies, and an Alexa fluor 488 or 568-conjugated secondary antibody was used as a secondary antibody. The coverslip was mounted using a mounting medium containing DAPI (VECTOR, Burlingame, Calif.). Images were obtained using a fluorescence microscope (Leica, Wetzlar, Germany).

Expression analysis of surface markers UEA-I (VECTOR), vWF (abcam), CD31, CD309, CD105, CD45 and CD34 was performed using Novocyte (Agilent, CA, USA) and analyzed with Novosoftware (Agilent).

For analysis of the response of BM-EPCs to TNF-oc, BM-EPCs (1.0 × 10⁵) were cultured in a 6-well plate coated with collagen type I, and added with 10 ng/ml TNF-α (PeproTech Inc, Rocky Hill, USA) and cultured for 24 hours, and then the expression levels of ICAM-1 (abcam) and VCAM-1 (Santa Cruz) were analyzed by Western blot.

### 1-2. ELISA assay

EPCs (2.0 × 10⁵ cells) were cultured in a 100 mm culture dish and an EPC-conditioned medium was collected on day 4. Then, the levels of HGF, TGF-β1, VEGF and PDGF-BB were measured according to the instructions of a Quantikine ELISA (R&D systems, Minneapolis, USA) manufacturer. Absorbance was measured at 450 nm using a microplate reader (Biotek, VT, USA).

### 1-3. Tube formation assay

Matrigel (Corning Inc, NY, USA) was treated to a µ-slide (Ibidi, Grafelfing, Germary) at 10 µl per well. EPCs (4.0 × 10³) or mixed cells of EPCs (4.0 × 10³) and MSCs (2.0 × 10³) were suspended in an α-MEM (GIBCO, NY, USA) medium containing 0.2% FBS, seeded on the Matrigel, and then cultured under treatment of VEGF, HGF and PDGF-BB (PeproTech) at concentrations of 0 to 40 ng/ml. Thereafter, images were obtained with a microscope (Leica).

### 1-4. Cell viability assay and 5-Bromo-2'-deoxyuridine (BrdU) cell labeling

For cell viability analysis, EPCs (1.0 × 10⁴ /well) were seeded in a 96-well plate coated with collagen type I, maintained in a starvation state for 10 hours in 0.2 % FBS-containing EBM-2, and then treated with PDGF-BB at 0, 10, 20 and 40 ng/ml for 24 hours, respectively. WST analysis was performed according to the manufacturer's instructions (Roche, Basel, Switzerland). In addition, absorbance was measured at 450 nm using a multi-plate reader (Biotek).

For BrdU cell labeling, EPCs (1.0 × 10⁴ /well) were cultured on a coverslip coated with collagen type I, treated with 20 ng/ml PDGF-BB in a fresh medium for 20 hours, and then added with 10 µM BrdU (Sigma Aldrich) and cultured for 4 hours. Then, the EPCs were immobilized using a methanol solvent for 10 minutes. Thereafter, the coverslip was treated with 2 N HCl for 1 hour at 37°C and neutralized with 0.1 M borate buffer, and then treated with primary antibodies to detect BrdU (Roche), treated with an FITC-conjugated secondary antibody (Invitrogen, California, USA) and cultured, and counterstained with PI (Sigma Aldrich) and then images were obtained with a fluorescence microscope (Leica).

### 1-5. Western blot

A BM-EPC lysate was obtained in a lysis buffer (Cell signaling, Danvers, MA) added with 2 mM PMSF (Sigma Aldrich). Protein concentrations were determined by BCA assay (Thermo Scientific, Waltham, Mass.). The EPC lysate and the conditioned medium were subjected to SDS-PAGE electrophoresis and transferred to a nitrocellulose membrane. Subsequently, primary antibodies were used to detect VCAM-1 (Santa Cruz), ICAM-1 (abcam), laminin-β1 (Santa Cruz), and α-tubulin (Sigma Aldrich), and an HRP binding-secondary antibody (BD, San Jose, CA, USA) was added and reacted. Thereafter, the antibody-reacted membrane was developed using EZ-Western Lumi Pico (Dogen, Seoul, Korea), signals were detected using a chemilluminator, and then quantified using ImageJ software.

### 1-6. Wound healing assay (Scratch assay)

EPC cell monolayers on a 6-well plate coated with collagen type I were maintained in a starved state for 18 hours in 0.2% FBS + EBM-2. Then, the cells were scraped with a 200 µl pipette tip, washed with 1x DPBS, and then cultured in a 0.2% FBS + α-MEM medium containing 0, 10, or 20 ng/ml of PDGF-BB. The wound healing process was observed under a microscope (Leica).

### 1-7. Statistical analysis

All data were expressed as mean ± standard deviations, and a P value < 0.05 was considered as a significant value (*p < 0.05, **p < 0.01, ***p < 0.001).

### <Experimental Example 2> Analysis of phenotypes and characteristics of Human BM mononuclear cell (MNC)-derived endothelial progenitor cells (EPCs)

Human bone marrow-endothelial progenitor cells (BM-EPCs) having colony formation were isolated from human bone marrow mononuclear cells, and colonies of endothelial progenitor cells (BM-EPCs) appeared to have a shape close to a spindle morphology rather than a cobblestone morphology (FIG. 1A). Immunofluorescence staining, ELISA, flow cytometry, and Matrigel tube formation assay were performed to confirm the phenotypes of the isolated endothelial progenitor cells. Lectin binding of UEA-1 as a common marker of endothelial lineage cells and expression of vWF markers were confirmed by double fluorescent staining (FIG. 1B).

Thereafter, major angiogenesis factors such as HGF, TGF-β1, VEGF, and PDGF-BB were measured in a BM-EPC-conditioned medium for each passage by an ELISA method. As a result, it was shown that the human bone marrow-endothelial progenitor cells (BM-EPCs) secreted HGF, TGF-β1 and VEGF, and it was shown that the expression levels decreased with passage, and PDGF-BB was not secreted in all cultures of passages 2 to 4 (FIG. 1C).

In addition, the expression levels of cell surface markers were measured by flow cytometry, but human BM-EPCs cultured for passages 1 to 3 had low expression of CD31 (< 2%), CD309 (< 3%), CD45 (< 2%) and CD34 (<1%), but had high expression of CD105 (> 95%) (FIG. 1D). In addition, it was confirmed that the BM-EPCs exhibited high UEA-I binding (> 99%) and vWF (> 99%) expression, as confirmed by fluorescence staining in subculturing once to three times. In addition, according to the Matrigel tube formation assay, BM-EPCs showed very low tube formation capacity up to subculturing three times, but showed tube formation capacity in subculturing 4 to 6 times (FIG. 1E).

In addition, the BM-EPCs showed very low tube formation capacity in subculturing three times, but when the BM-EPCs and the BM-MSCs were mixed at a cell number ratio of 2 : 1, a well-formed vascular network was confirmed (FIG. 1F).

Therefore, through these results, the present inventors confirmed that human bone marrow mononuclear cell-derived endothelial progenitor cells (BM-EPCs) had colony-forming ability, and was a cell group which was very rapidly divided as the time for cells to double by 3rd subculturing (P3) was about 1.5 days (FIG. 2). In addition, it was shown that the BM-EPCs may be divided very rapidly in early passages, secreted angiogenesis factors such as VEGF, HGF, and TGF-β1, and further increased vasculogenesis when cultured with BM-MSC cells. Therefore, these results indicate that the mixed cells by co-culture of BM-EPCs and BM-MSCs may be usefully used as a cell therapeutic agent for treating various ischemic and chronic vascular diseases.

### <Experimental Example 3> Confirmation of increased expression of inflammatory adhesion factors in endothelial progenitor cells by TNF-α treatment

TNF-α was known to up-regulate the expression of pro-inflammatory adhesion factors such as ICAM-1 and VCAM-1 in endothelial cells. Accordingly, the present inventors analyzed by Western blot analysis whether the human bone marrow-endothelial progenitor cells (BM-EPCs) of the present invention may induce the expression of pro-inflammatory factors when treated with TNF-oc.

As a result, it was shown that the BM-EPCs did not express ICAM-1, but when treated with TNF-α, ICAM-1 expression increased significantly (FIG. 3A). In addition, it was shown that the BM-EPCs significantly upregulated VCAM-1 expression upon TNF-α treatment (FIGS. 3B and 3C).

Through these results, the present inventors found that the BM-EPCs may induce an inflammatory response when treated with TNF-α, and as a result, the BM-EPCs of the present invention had characteristics of endothelial cells.

### <Experimental Example 4> Analysis of effect of increasing vasculogenesis of human bone marrow-endothelial progenitor cells (BM-EPCs) according to treatment of angiogenesis factors

To confirm whether VEGF, HGF, and PDGF-BB, known as angiogenesis-stimulating factors of endothelial cells, affected the vasculogenesis capacity of endothelial progenitor cells, endothelial progenitor cells (4.0 × 10³ cells/well) were dissolved in an α-MEM medium containing 0.2% FBS, and dispensed in Matrigel of a µ-slide containing VEGF, HGF, and PDGF-BB at each concentration of 0, 10, 20, and 40 ng/ml, and then the vasculogenesis was observed by the number of meshes, the number of junctions, the number of segments, and the total length between two junctions.

As a result, as illustrated in FIG. 4, it was shown that VEGF did not show a significant difference in the vasculogenesis capacity of endothelial progenitor cells, but PDGF-BB greatly increased the vasculogenesis capacity of endothelial progenitor cells in a concentration-dependent manner. In addition, even in an HGF-treated group, it was shown that the vasculogenesis capacity of endothelial progenitor cells was increased, but the increase in vasculogenesis capacity did not reach that of the PDGF-BB-treated group.

Accordingly, the present inventors have found that treatment with PDGF-BB was very useful as a cell activation method capable of effectively increasing the vasculogenesis capacity of endothelial progenitor cells.

In addition, through the previous Examples, it was confirmed that PDGF-BB was not secreted from BM-EPCs, but it was confirmed that PDGF-BB was present in an abundant amount under wound conditions. Accordingly, the present inventors treated BM-EPCs subcultured three times with PDGF-BB at different concentrations of 0, 10, 20, and 40 ng/ml, and then observed the tube-forming capacity while continuously monitoring for 18 hours.

As a result, as illustrated in FIG. 5, it was confirmed that the number of meshes, the number of junctions, the number of segments, and the total length between two junctions of human BM-EPCs were increased in a concentration-dependent manner of PDGF-BB treatment, thus the vasculogenesis was increased.

### <Experimental Example 5> Cell response analysis of bone marrow-endothelial progenitor cells (BM-EPCs) according to PDGF-BB treatment

### 5-1. Effect on cell migration of endothelial progenitor cells

In order to analyze a change in migration capacity of endothelial progenitor cells according to PDGF-BB treatment, a gap filling rate was measured after scratch damage to endothelial progenitor cells. Specifically, endothelial progenitor cells (1.0 × 10⁵ cells/well) were cultured in 6 well and starved for 18 h, and then cell monolayers of the endothelial progenitor cells were scraped with a 200 µl tip and then washed twice with PBS. Thereafter, PDGF-BB was treated in an α-MEM medium containing 0.2% FBS at different concentrations of 0, 10, and 20 ng/ml, and then a wound closure process was measured.

As a result, as illustrated in FIG. 6, cell migration of the endothelial progenitor cells was increased in a group treated with PDGF-BB compared to a group not treated with PDGF-BB.

### 5-2. Effect on cell viability of endothelial progenitor cells

Changes in viability of endothelial progenitor cells by passage according to PDGF-BB treatment were analyzed. To this end, the endothelial progenitor cells (1.0 × 10⁴ cells/well) were cultured in 96 well, maintained in a starved state for 18 hours, treated with PDGF-BB for 24 h at each concentration of 0, 10, 20, and 40 ng/ml, and then treated with a WST-1 solution, and thereafter, cell viability was analyzed by measuring absorbance at 450 nm.

As a result, as illustrated in FIG. 7, it was shown that in endothelial progenitor cells of P2 and P3 passages, cell viability increased in proportion to the treatment concentration of PDGF-BB, but no significant difference was confirmed by PDGF-BB treatment in P4.

### 5-3. Effect on cell proliferation of endothelial progenitor cells

Changes in cell proliferation of endothelial progenitor cells according to PDGF-BB treatment were analyzed. To this end, endothelial progenitor cells (1.0 × 10⁴ cells) were cultured on a coverslip in 24 well, maintained in a starved state for 18 hours, and then treated with PDGF-BB at different concentrations of 0 and 20 ng/ml for 24 hours. In addition, at 20 hours after PDGF-BB treatment, 10 µM BrdU was treated for 4 hours, and the number of cells entering S-phase was measured. In addition, endothelial cells were immobilized with 3.7% formaldehyde, permeabilized, and sequentially treated with primary and secondary antibodies, and then subjected to nuclear staining with PI staining. The degree of cell proliferation was analyzed by counting BrdU-positive cells among PI-positive cells under a fluorescence microscope.

As a result, as illustrated in FIG. 8, it was confirmed that the number of endothelial progenitor cells was increased in a group treated with PDGF-BB compared to a group not treated with PDGF-BB and thus cell proliferation occurred.

### 5-4. Effect on expression of laminin β1, VEGFR2 and PDGFRβ

The expression level of laminin β1, an extra-matrix protein of endothelial progenitor cells according to PDGF-BB treatment was analyzed by fluorescence staining and Western blotting. To this end, endothelial progenitor cells (1.0 × 10⁴ cells) were cultured on a coverslip in 24 well, maintained in a starved state for 18 hours, and then treated with PDGF-BB at different concentrations of 0, 10, and 20 ng/ml for 24 hours. The endothelial cells were immobilized with 3.7% formaldehyde, permeabilized, treated with a primary antibody against laminin β1, then treated with a secondary antibody, and then stained with DAPI to analyze the expression level of laminin β1.

As a result, as illustrated in FIG. 9, the expression of laminin β1 in the endothelial progenitor cells was increased in proportion to the treatment concentration of PDGF-BB. These results were identical to the results of Western blotting on the culture medium of the endothelial progenitor cells.

In addition, in order to analyze changes in expression of VEGFR2 and PDGFRβ in endothelial progenitor cells according to PDGF-BB treatment, the analysis was performed in the same manner as described above using antibodies against VEGFR2 and PDGFRβ. As a result, as illustrated in FIG. 10, it was shown that the expression of VEGFR2 was not observed in the endothelial progenitor cells, and treatment with PDGF-BB did not affect VEGFR2 expression.

Meanwhile, it was shown that internalization of PDGFRβ was increased in proportion to the treatment concentration of PDGF-BB. As the results confirmed above, this is expected to be related to an increase in cell migration, an increase in cell viability, an increase in cell proliferation, and an increase in the expression of laminin β1, an extra-matrix protein, due to the treatment of PDGF-BB.

### <Experimental Example 6> Co-culture of bone marrow-endothelial progenitor cells (BM-EPCs) and mesenchymal stem cells and confirmation of effective increase in vasculogenesis by PDGF-BB treatment

The present inventors confirmed through the experiments of Examples that when BM-EPCs were treated with PDGF-BB, there was an increased activity of the vasculogenesis. Accordingly, as a method for further increasing the vasculogenesis of BM-EPCs, the present inventors performed an experiment for confirming whether to effectively increase the vasculogenesis when co-culturing BM-EPCs with mesenchymal stem cells and then treating PDGF-BB therein.

To this end, endothelial progenitor cells (4.0 × 10³ cells/well) and mesenchymal stem cells (2.0 × 10³ cells/well) were suspended in an α-MEM medium containing 0.2% FBS at a 2 : 1 ratio (ratio of cell numbers), treated with PDGF-BB for each concentration of 0, 10, 20, and 40 ng/ml, and then these cell cultures were dispensed on Matrigel of a µ-slide to observe the degree of vasculogenesis.

As a result, as illustrated in FIG. 11, it was shown that in the case of a group co-cultured with endothelial progenitor cells and mesenchymal stem cells, the vasculogenesis increased compared to a group in which the endothelial progenitor cells were cultured alone. In addition, it was shown that when PDGF-BB was treated in the group co-cultured with the endothelial progenitor cells and the mesenchymal stem cells, the vasculogenesis was further increased in proportion to the treatment concentration.

Accordingly, through these results, the present inventors found that mixed cells including endothelial progenitor cells and mesenchymal stem cells can be used as a combined stem cell therapeutic agent capable of increasing vasculogenesis and increasing revascularization, and particularly, when these mixed cells were treated with PDGF-BB, vasculogenesis could be further increased.

Therefore, the mixed cells of the endothelial progenitor cells and the mesenchymal stem cells treated with PDGF-BB designed in the present invention may be used as a novel cell therapeutic agent for treating ischemic diseases caused by a decrease in vasculogenesis.

Hereinabove, the present invention has been described with reference to preferred embodiments thereof. It will be understood to those skilled in the art that the present invention may be implemented as a modified form without departing from an essential characteristic of the present invention. Therefore, the disclosed embodiments should be considered in an illustrative viewpoint rather than a restrictive viewpoint. The scope of the present invention is illustrated by the appended claims rather than by the foregoing description, and all differences within the scope of equivalents thereof should be construed as being included in the present invention.

The present invention was supported and performed by advanced medical technology development (R&D) research project of the Ministry of Health and Welfare (development of complex stem cell therapeutic agent of autologous bone marrow-mesenchymal stem cells and endothelial progenitor cells, project identification number: 1465032804, project detail number: HI18C1492010021).

## Claims

1. A method for preparing endothelial progenitor cells having increased vasculogenesis, comprising treating and culturing isolated endothelial progenitor cells (EPCs) with platelet-derived growth factor-BB (PDGF-BB).

2. The method for preparing endothelial progenitor cells having increased vasculogenesis of claim 1, wherein the endothelial progenitor cells are human bone marrow-endothelial progenitor cells (BM-EPCs).

3. The method for preparing endothelial progenitor cells having increased vasculogenesis of claim 1, wherein the endothelial progenitor cells treated with the PDGF-BB have increased cell migration, increased cell viability, increased cell proliferation, and increased expression of an extra-matrix protein laminin β1.

4. A composition for stimulating vasculogenesis for treating anangioplasia-related diseases comprising isolated endothelial progenitor cells (EPCs), mesenchymal stem cells (MSCs), and platelet-derived growth factor-BB (PDGF-BB) as active ingredients.

5. The composition for stimulating vasculogenesis for treating anangioplasia-related diseases of claim 4, wherein the endothelial progenitor cells are human bone marrow-endothelial progenitor cells (BM-EPCs).

6. A method for preparing a cell therapeutic agent for treating anangioplasia-related diseases comprising mixing isolated endothelial progenitor cells (EPCs), mesenchymal stem cells (MSCs), and platelet-derived growth factor-BB (PDGF-BB).

7. The method for preparing a cell therapeutic agent for treating anangioplasia-related diseases of claim 6, wherein the isolated endothelial progenitor cells and the mesenchymal stem cells are mixed in a cell number ratio of 1 : 1 to 2 : 1.

8. The method for preparing a cell therapeutic agent for treating anangioplasia-related diseases of claim 6, wherein the endothelial progenitor cells are human bone marrow-endothelial progenitor cells, and
the mesenchymal stem cells are human bone marrow-mesenchymal stem cells.

9. The method for preparing a cell therapeutic agent for treating anangioplasia-related diseases of claim 6, wherein the anangioplasia-related disease is selected from the group consisting of ischemic disease, diabetic ulcer, gangrene, obstructive vascular disease, cardiovascular disease and ischemia.

10. The method for preparing a cell therapeutic agent for treating anangioplasia-related diseases of claim 9, wherein the ischemic disease is selected from the group consisting of ischemic myocardial infarction, ischemic heart disease, ischemic vascular disease, ischemic eye disease, ischemic renal failure, ischemic retinopathy, ischemic stroke and ischemic lower limb disease.

11. A cell therapeutic agent for treating anangioplasia-related diseases, prepared by the method of any one of claims 6 to 10.

12. The cell therapeutic agent for treating anangioplasia-related diseases of claim 11, wherein the cell therapeutic agent is mixed with bone marrow-endothelial progenitor cells and bone marrow-mesenchymal stem cells in a cell number ratio of 1 : 1 to 2 : 1 and has vasculogenesis.

13. The cell therapeutic agent for treating anangioplasia-related diseases of claim 11, wherein the cell therapeutic agent is for intramedullary administration, intravenous administration, subcutaneous administration, intramuscular administration or intraperitoneal administration.

14. The cell therapeutic agent for treating anangioplasia-related diseases of claim 11, wherein the anangioplasia-related disease is selected from the group consisting of ischemic disease, diabetic ulcer, gangrene, obstructive vascular disease, cardiovascular disease and ischemia.

15. The cell therapeutic agent for treating anangioplasia-related diseases of claim 14, wherein the ischemic disease is selected from the group consisting of ischemic myocardial infarction, ischemic heart disease, ischemic vascular disease, ischemic eye disease, ischemic renal failure, ischemic retinopathy, ischemic stroke and ischemic lower limb disease.

16. A method for preventing or treating anangioplasia-related diseases, comprising administering the composition for stimulating vasculogenesis of claim 4 or 5 to a subject.

17. A method for preventing or treating anangioplasia-related diseases, comprising administering the cell therapeutic agent prepared by the preparing method of any one selected from claims 6 to 10 to a subject.
